# EUROPÄISCHE PATENTSCHRIFT

(11) **EP 1 278 210 B1**
(45) Veröffentlichungstag und Bekanntmachung des Hinweises auf die Patenterteilung: **04.08.2010**
(21) Anmeldenummer: 02013837.6
(22) Anmeldetag: 21.06.2002
(51) Int. Cl.: G21K 4/00, H04N 5/321

(54) **Kombiniertes Radiographie- und Fluoroskopiegerät**
Combined radiographic and fluoroscopic apparatus
Dispositif combiné de radiographie et de fluoroscopie

(30) Priorität: 26.06.2001 DE 10130616
(43) Veröffentlichungstag der Anmeldung: 22.01.2003
(73) Patentinhaber: SIEMENS AKTIENGESELLSCHAFT, 80333 München (DE)
(72) Erfinder: Freudenberger, Joerg, Dr., 91083 Baiersdorf (DE); Fuchs, Manfred, 90459 Nürnberg (DE); Hell, Erich, Dr., 91054 Erlangen (DE); Mattern, Detlef, Dr., 91056 Erlangen (DE)

(56) Entgegenhaltungen:
- DE-A- 19 914 217
- DE-C- 19 946 736
- GB-A- 2 014 420
- GB-A- 2 096 440

## Beschreibung

Die Erfindung bezieht sich auf ein kombiniertes Radiographie- und Fluoroskopiegerät mit einer von der hochenergetischen Objektstrahlung bestrahlten Leuchtstoffplatte, deren von einem digitalen Bildempfänger aufgenommene Fluoreszenzstrahlung in Echtzeit auf einem Bildschirm dargestellt wird.

Bisher wurden zur Ausnutzung der Vorteile von Radiographie und Fluoroskopie entweder zwei Geräte benötigt, oder - bei Verwendung eines Gerätes - wurden nicht alle Vorteile des jeweiligen Verfahrens ausgenutzt. Eine typische Variante eines bislang bekannten Kombinationsgerätes ist ein Röntgenbildverstärkersystem mit einer zusätzlichen Filmkassette. Der Bildverstärker ermöglicht Fluoroskopie.

In der Filmkassette können entweder Film-Foliensysteme oder Speicherfolien eingesetzt werden. In beiden Fällen müssen Bilder für Radiographie also manuell entwickelt werden.

Die DE 199 46 736 C1 betrifft eine Röntgendiagnostikeinrichtung mit einem Speicherleuchtschirm für die latente Speicherung des jeweiligen Röntgenstrahlenbildes, mit einer Auslesevorrichtung, bei der zur Bildwiedergabe der Speicherleuchtschirm durch eine Abtastung mittels einer Strahlenquelle zum Leuchten angeregt wird, wobei die von dem Speicherleuchtschirm emittierte Strahlung von einem Detektor erfasst und an ein Bildwiedergabesystem weitergeleitet wird. Es ist hierbei ein erster Betriebsmodus, in dem die Röntgenaufnahmevorrichtung Röntgenbilder mit einer ersten Dosis erstellt sowie die Auslesevorrichtung eine schnelle Auslesung der Bildfläche des Speicherleüchtschirms mit geringer Auflösung durchführt und ein zweiter Betriebsmodus, in dem die Röntgenaufnahmevorrichtung Röntgenbilder mit einer zweiten, gegenüber der ersten höheren Dosis in einem eingeschränkten Bereich erstellt sowie dieser Teil der Bildfläche des Speicherleuchtschirmes mit hoher Auflösung ausgelesen wird, vorgesehen.

Aus der GB-A-2 014 420 ist eine Röntgenuntersuchungsanordnung mit einem Röntgenbildverstärker mit einem faseroptischen Austrittsfenster, einem optischen Bildübertragungssystem und einem Fernsehkreis, wobei ein Blatt- oder Rollenfilm zwischen das optische System und dem Röntgenbildverstärker einführbar und an das Austrittsfenster des Röntgenbildverstärkers andrückbar ist, bekannt. Hierbei werden ausdrücklich geringe Strahlendosen verwendet.

Der Erfindung liegt daher die Aufgabe zugrunde, ein kombiniertes Radiographie- und Fluoroskopiegerät zu schaffen, welches bei einfachem Aufbau eine besonders einfache und günstige Anwendung der beiden Verfahren unter Beibehaltung aller ihrer Vorteile und Stärken gestattet.

Zur Lösung dieser Aufgabe ist erfindungsgemäß vorgesehen, dass ein Fluoroskopiegerät der Eingangs beschriebenen Art in der Weise aufgebaut ist, dass die Leuchtstoffplatte als Speicherleuchtstoffplatte ausgebildet ist, der ein Scanner zugeordnet ist, der für den Radiographiemodus unter Einstrahlung von Licht ein Abtasten des latenten Speicherenergiebildes ermöglicht.

Die Erfindung geht dabei von der Erkenntnis aus, dass bei geeigneten Speicherleuchtstoffen, beispielsweise CsBr:Eu, also einem Europium dotierten Cäsiumbromid, oder BaFlBr:Eu, einem Europium dotierten Bariumfluorbromid, beim Einfall der Objektstrahlung, also der hinter einem durchstrahlten Objekt anfallenden Röntgenstrahlung, sowohl eine direkte Lichtemission erfolgt, als auch eine teilweise Speicherung der Objektstrahlung in der Speicherleuchtstoffschicht. Die direkte Emission kann für die Fluoroskopie verwendet werden, während die gespeicherte Information für die Radiographie ausgenutzt wird.

Der digitale Bildempfänger, dem bevorzugt ein Bildverstärker vorgeschaltet sein kann, kann beispielsweise eine CCD-Einheit, ein Photodiodenarray oder dergleichen umfassen, wobei im Hinblick auf die wahlweise Verwendung des erfindungsgemäßen Kombinationsgerätes für Fluoroskopie oder für Radiographie der Speicherleuchtstoffplatte eine Beleuchtungseinrichtung, insbesondere eine starke Blitzlichtquelle, zur Regenerierung für den Radiographiemodus zugeordnet sein soll.

Die Ausbildung kann dabei im Einzelnen unterschiedlich getroffen sein. So kann beispielsweise der Scanner hinter der Speicherleuchtstoffplatte und wiederum hinter diesem ein Spiegel zum Ausblenden der Fluoreszenzstrahlung auf den seitlich außerhalb des Primärstrahls angeordneten Bildempfänger vorgesehen sein. Anstelle dieser Anordnung kann auch ein vor der Speicherleuchtstoffplatte angeordneter Scanner verwendet werden, wobei dann die Rückseite der Speicherleuchtstoffplatte über eine Glasfaseroptik an den Bildverstärker angekoppelt sein kann. In beiden Fällen ist der Scanner bevorzugt ein mit einem feinen Abtastlichtstrahl arbeitender Abtastscanner, der in Abhängigkeit von der Feinheit des Abtaststrahls eine entsprechende Auflösungsempfindlichkeit und damit eine hohe Bildqualität gewährleistet.

Anstelle der vorstehend beschriebenen Ausbildung mit einem Scanner mit Abtastlichtstrahl und Empfangsdioden der über die Fläche der Speicherleuchtstoffschicht zum Abtasten verschoben wird, kann erfindungsgemäß die Ausbildung auch so getroffen sein, dass die beidseits von elektronischen Blenden flankierte Speicherleuchtstoffplatte zwischen einem - von der Objektstrahlung durchstrahlten - Leuchtdioden-Array und einem Fotodioden-Array als Bildempfänger angeordnet ist.

Dabei hat es sich als besonders zweckmäßig erwiesen, wenn dem Fotodioden-Array ein Farbfilter zum Ausblenden der Austaststrahlung des Leuchtdioden-Arrays vorgeschaltet ist, sodass die Austaststrahlung des Leuchtdioden-Arrays, die meist im infraroten Bereich liegt, überhaupt nicht auf den Fotodioden-Array als Bildverstärker gelangen kann, der nur die kurzwelligere Fluoreszenzstrahlung empfängt.

Die Leuchtdioden werden in diesem Fall sowohl zur Löschung, also zur Regenerierung der Speicherleuchtstoffplatte vor dem Radiographiemodus, als auch zur Auslesung des Speicherleuchtstoffs verwendet. Die elektronischen Blenden erlauben es dabei im Auslesemodus das Licht einer Fotodiode auf ein Pixel des Detektors zu begrenzen und darüber hinaus auch nur einen örtlich begrenzten Teil des Speicherleuchtstoffs durch die Fotodiode zu beleuchten.

Im Regenerierungsmodus ist die untere, also die dem Fotodioden-Array zugekehrte elektronische Blende geschlossen und nur die obere durchlässig. Auf diese Art und Weise kann die im Speicherleuchtstoff gespeicherte Information durch Belichtung mittels der Leuchtdioden wieder gelöscht werden.

Weitere Vorteile, Merkmale und Einzelheiten der Erfindung ergeben sich aus der nachfolgenden Beschreibung einiger Ausführungsbeispiele sowie anhand der Zeichnung. Dabei zeigen:
- Fig. 1: Eine schematische Darstellung eines ersten Ausfüh- rungsbeispiels mit einem Scanner und einer digitalen Kamera,
- Fig. 2: eine schematische Ausführung eines erfindungsgemäßen kombinierten Fluoroskopie- und Radiographiegerätes mit Bildverstärker und Faseroptik und
- Fig. 3: ein drittes wiederum schematisch wiedergegebenes Ausführungsbeispiel eines erfindungsgemäßen Kombina- tionsgerätes unter Verwendung von Leucht- und Foto- dioden-Arrays zum Auslesen und als Bildempfänger.

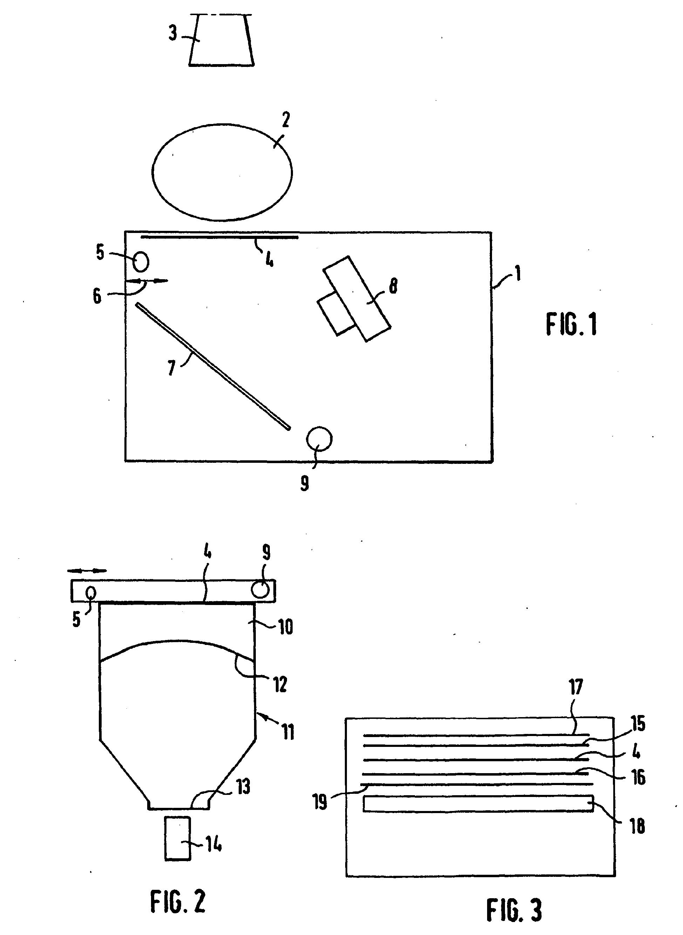

In **Fig. 1** ist innerhalb einer lichtdichten Ummantelung 1 oben unterhalb eines nur schematisch als ovaler Umriss angedeuteten Patienten 2, über dem eine Röntgenröhre 3 angeordnet ist, eine Speicherleuchtstoffplatte 4 angeordnet, die mit einem geeigneten Speicherleuchtstoff beschichtet ist, der sowohl die direkte Lichtemission für Fluoroskopiezwecke ausnützen kann, als auch die angeregten Speicherzustände des Speicherleuchtstoffs für einen Radiographiemodus. Bei 5 ist schematisch ein Scanner für hochauflösende Radiographie angedeutet, der in Richtung des Doppelpfeils 6 verschiebbar ist und der die Ausstrahlung eines feinen Lichtstrahls auf die Speicherleuchtstoffplatte 4 und einen Empfang der in einem anderen Wellenbereich zurückgestrahlten Speicherstrahlung ermöglicht. Im Strahlengang hinter der Speicherleuchtstoffplatte 4 ist ein Spiegel 7 angeordnet, der die Fluoreszenzstrahlung der Speicherleuchtstoffplatte, die zeitgleich mit der Bestrahlung durch die abgekürzt als Objektstrahlung bezeichnete Strahlung nach Durchstrahlung des Patienten 2 im Speicherleuchtstoff ausgelöst wird, zur Seite ablenkt und einer digitalen Kamera 8 zuführt, die auf diese Art und Weise außerhalb des Primärstrahls angeordnet sein kann. Diese digitale Kamera, die für die Fluoroskopie dient, kann über eine spezielle Optik-Einheit an die Speicherleuchtstoffplatte gekoppelt sein, die aber - weil an sich bekannt - im Rahmen der vorliegenden Anmeldung nicht näher beschrieben werden soll. Bei 9 ist eine Blitzlampe zum Löschen der Informationen der Speicherleuchtstoffplatte 4 angeordnet, die jedes Mal dann betätigt wird, wenn anschließend in den Radiographiemodos übergegangen werden soll.

Bei dem in **Fig. 2** schematisch angedeuteten Ausführungsbeispiel liegt der Scanner 5 vor der Speicherleuchtstoffplatte 4, wobei darüber dann wiederum, wie in Fig. 1, der Patient und darüber die Röntgenröhre zu denken ist. Diese Versetzung des Scanners für die Radiographie auf die gegenüberliegende Seite der Speicherleuchtstoffplatte 4 ermöglicht eine direkte Ankoppelung dieser Speicherleuchtstoffplatte 4 über eine Glasfaseroptik 10 an ein Röntgenbildverstärkersystem 11 mit einer Fotokathode 12, einer dieser nachgeschalteten, nicht im Einzelnen dargestellten Elektronenoptik und einem Ausgangsschirm 13, hinter dem sich wiederum eine digitale Kamera 14 befindet. Diese dient, wie beim Ausführungsbeispiel nach Fig. 1, für Fluoroskopie.

Beim Ausführungsbeispiel nach **Fig. 3** ist die Speicherleuchtstoffplatte 4 zwischen zwei elektronischen Blenden 15 und 16 angeordnet. Über der oberen elektronischen Blende 15, also direkt im Einfallsbereich der Objektstrahlung, ist ein Leuchtdioden-Array 17 vorgesehen, das von der Objektstrahlung (Röntgenstrahlung) problemlos durchsetzt wird, um auf der Speicherleuchtstoffplatte einerseits Fluoreszenzstrahlung auszulösen und andererseits teilweise als latentes Speicherenergiebild abgespeichert zu werden. Zwischen der unteren elektronischen Blende 16 und einem Flat-Panel-Detektor, vorzugsweise in Form eines Fotodioden-Arrays 18, ist ein Farbfilter 19 angeordnet, der die meist rote Auslesestrahlung des Leuchtdioden-Arrays 17 ausblendet und nur die üblicherweise blaue Fluoreszenzstrahlung der Speicherleuchtstoffplatte 14 passieren lässt.

Die Leuchtdioden des Leuchtdioden-Arrays 17 werden sowohl zum Löschen der Speicherleuchtstoffplatte 4, als auch zum Auslesen des Speicherleuchtstoffs verwendet. Die elektronischen Blenden 15, 16, beispielsweise gekreuzte Polarisationsfilter mit einer Zwischenschicht zum Drehen der Polarisationsebene, erlauben es im Auslesemodus das Licht einer Fotodiode auf ein Pixel des Detektors zu begrenzen und nur einen örtlich begrenzten Teil des Speicherleuchtstoffs durch die Fotodiode zu beleuchten.

Im Regenerierungsmodus ist die untere Blende 16 geschlossen und die obere elektronische Blende 15 lichtdurchlässig. So kann die im Speicherleuchtstoff gespeicherte Information durch Belichtung mittels der Leuchtdioden gelöscht werden.

## Patentansprüche

1. Kombiniertes Radiographie- und Fluoroskopiegerät mit einer von der hochenergetischen Objektstrahlung bestrahlten Leuchtstoffplatte, deren von einem digitalen Bildempfänger (8, 14) aufgenommene Fluoreszenzstrahlung in Echtzeit auf einem Bildschirm dargestellt wird, **dadurch gekennzeichnet, dass** die Leuchtstoffplatte als Speicherleuchtstoffplatte (4) ausgebildet ist, der ein Scanner (5, 17) zugeordnet ist, der für den Radiographiemodus unter Einstrahlung von Licht ein Abtasten des latenten Speicherenergiebildes ermöglicht.

2. Kombiniertes Radiographie- und Fluoroskopiegerät nach Anspruch 1, **dadurch gekennzeichnet, dass** dem Bildempfänger (8, 14) ein Bildverstärker (11) vorgeschaltet ist.

3. Kombiniertes Radiographie- und Fluoroskopiegerät nach Anspruch 1 oder 2, **dadurch gekennzeichnet, dass** der Bildempfänger (8, 14) eine CCD, ein Photodioden-Array (18) oder dergleichen umfasst.

4. Kombiniertes Radiographie- und Fluoroskopiegerät nach einem der Ansprüche 1 bis 3, **dadurch gekennzeichnet, dass** der Speicherleuchtstoffplatte (4) eine Beleuchtungsvorrichtung, insbesondere eine starke Blitzlichtquelle (9) zur Regenerierung für den Radiographiemodus zugeordnet ist.

5. Kombiniertes Radiographie- und Fluoroskopiegerät nach einem der Ansprüche 1 bis 4, **dadurch gekennzeichnet, dass** der Scanner (5) hinter der Speicherleuchtstoffplatte (4) und nach dem Scanner (5) ein Spiegel (7) zum Ausblenden der Fluoreszenzstrahlung auf den seitlich außerhalb des Primärstrahls angeordnetem Bildempfänger (8) vorgesehen ist.

6. Kombiniertes Radiographie- und Fluoroskopiegerät nach einem der Ansprüche 1 bis 4, **dadurch gekennzeichnet, dass** der Scanner (5) vor der Speicherleuchtstoffplatte (4) angeordnet ist, die auf der Rückseite über eine Glasfaseroptik (10) an den Bildverstärker (11) angekoppelt ist.

7. Kombiniertes Radiographie- und Fluoroskopiegerät nach einem der Ansprüche 1 bis 6, **dadurch gekennzeichnet, dass** der Scanner (5) ein mit einem feinen Abtastlichtstrahl arbeitender Abtastscanner ist.

8. Kombiniertes Radiographie- und Fluoroskopiegerät nach einem der Ansprüche 1 bis 4, **dadurch gekennzeichnet, dass** die beidseits von elektronischen Blenden (15, 16) flankierte Speicherleuchtstoffplatte (4) zwischen einem - von der Objektstrahlung durchstrahlten - Leuchtdioden-Array (17) und einem Photodioden-Array (18) als Bildempfänger angeordnet ist.

9. Kombiniertes Radiographie- und Fluoroskopiegerät nach Anspruch 8, **dadurch gekennzeichnet, dass** dem Photodioden-Array (18) ein Farbfilter (19) zum Ausblenden der Austaststrahlung des Leuchtdioden-Arrays (17) vorgeschaltet ist.

## Claims

1. Combined radiographic and fluoroscopic apparatus having a fluorescent plate subjected to high energy object radiation and whose fluorescent radiation acquired by a digital image receiver (8, 14) is shown on a screen in real time, **characterized in that** the fluorescent plate is embodied as a storage fluorescent plate (4) associated with a scanner (5, 17) that enables scanning of the latent storage energy image with incident light for radiography mode.

2. Combined radiographic and fluoroscopic apparatus according to claim 1, **characterized in that** an image amplifier (11) is connected upstream of the image receiver (8, 14).

3. Combined radiographic and fluoroscopic apparatus according to claim 1 or 2, **characterized in that** the image receiver (8, 14) comprises a CCD, a photodiode array (18) or the like.

4. Combined radiographic and fluoroscopic apparatus according to one of claims 1 to 3, **characterized in that** the storage fluorescent plate (4) is assigned an illumination device, especially a strong flash source (9) for regeneration for the radiography mode.

5. Combined radiographic and fluoroscopic apparatus according to one of claims 1 to 4, **characterized in that** the scanner (5) is provided behind the storage fluorescent plate (4) and a mirror is provided after the scanner on the image receiver (8) arranged laterally outside the primary beam for filtering out the fluorescence radiation.

6. Combined radiographic and fluoroscopic apparatus according to one of claims 1 to 4, **characterized in that** the scanner (5) is arranged before the storage fluorescent plate (4) which is coupled at the rear via a glass fiber optic (10) to the image amplifier (11).

7. Combined radiographic and fluoroscopic apparatus according to one of claims 1 to 6, **characterized in that** the scanner (5) is a sampling scanner working with a fine sampling light beam.

8. Combined radiographic and fluoroscopic apparatus according to one of claims 1 to 4, **characterized in that** the storage fluorescent plate (4) flanked on both sides by electronic shutters (15, 16) is arranged between a light emitting diode array (17) - through which the object radiation passes - and a photodiode array (18) as image receiver.

9. Combined radiographic and fluoroscopic apparatus according to claim 8, **characterized in that** a colour filter (19) for filtering out the blanking radiation of the light emitting diode array (17) is connected upstream from the photodiode array (18).

## Revendications

1. Appareil combiné de radiographie et de fluoroscopie comprenant une plaque luminescente qui est exposée au rayonnement objet de grande énergie et dont le rayonnement de fluorescence absorbé par un récepteur ( 8, 14 ) d'images numériques est représenté en temps réel sur un écran, **caractérisé en ce que** la plaque luminescente est constituée sous la forme d'une plaque ( 4 ) luminescente à accumulation, à laquelle est affecté un lecteur ( 5, 17 ) qui rend possible, pour le mode de radiographie avec incidence de lumière, un balayage de l'image d'énergie accumulée latente.

2. Appareil combiné de radiographie et de fluoroscopie suivant la revendication 1, **caractérisé en ce qu'**un amplificateur ( 11 ) de luminance est monté en amont du récepteur ( 8, 14 ) d'images.

3. Appareil combiné de radiographie et de fluoroscopie suivant la revendication 1 ou 2, **caractérisé en ce que** le récepteur ( 8, 14 ) récepteur d'images comprend un CCD, une matrice ( 18 ) de photodiodes ou analogue.

4. Appareil combiné de radiographie et de fluoroscopie suivant l'une des revendications 1 à 3, **caractérisé en ce qu'**un dispositif d'éclairage, notamment une source ( 9 ) intense de lumière à éclair, est affectée à la plaque ( 4 ) luminescente à accumulation pour la régénération pour le mode d'un radiographie.

5. Appareil combiné de radiographie et de fluoroscopie suivant l'une des revendications 1 à 4, **caractérisé en ce que** le lecteur ( 5 ) est prévu derrière la plaque ( 4 ) luminescente à accumulation et, après le lecteur ( 5 ), est prévu un miroir ( 7 ) pour supprimer le rayonnement de fluorescence sur le récepteur ( 8 ) d'images disposé latéralement à l'extérieur du faisceau primaire.

6. Appareil combiné de radiographie et de fluoroscopie suivant l'une des revendications 1 à 4, **caractérisé en ce que** le lecteur ( 5 ) est disposé devant la plaque ( 4 ) luminescente à accumulation, qui est couplée du côté arrière à l'amplificateur ( 1 ) de luminance par une optique ( 10 ) à fibre de verre.

7. Appareil combiné de radiographie et de fluoroscopie suivant l'une des revendications 1 à 6, **caractérisé en ce que** le lecteur ( 5 ) est un lecteur à balayage fonctionnant avec un fin faisceau de lumière de balayage.

8. Appareil combiné de radiographie et de fluoroscopie suivant l'une des revendications 1 à 4, **caractérisé en ce que** la plaque ( 4 ) luminescente à accumulation, flanquée des deux côtés de caches ( 15, 16 ) électroniques, est disposée entre une matrice ( 17 ) de diodes électroluminescentes traversée par le rayonnement objet et une matrice ( 18 ) de photodiodes en tant que récepteur d'images.

9. Appareil combiné de radiographie et de fluoroscopie suivant la revendication 8, **caractérisé en ce qu'**un filtre ( 19 ) coloré est disposé en amont de la matrice ( 18 ) de photodiodes, pour supprimer le rayonnement de suppression de la matrice ( 17 ) de diodes électroluminescentes.
